# EUROPEAN PATENT SPECIFICATION

(11) **EP 2 440 213 B1**
(45) Date of publication and mention of the grant of the patent: **06.11.2013**
(21) Application number: 10740275.2
(22) Date of filing: 02.06.2010
(51) Int. Cl.: A61K 31/7032, A61K 45/06, A61K 36/81, A61K 9/00, A61P 1/14, A61P 43/00, A61K 47/02, A61K 47/44

(54) **Use of 1,25-dihydroxyvitamine D3 glycoside in lactating animals**
Verwendung von 1,25-Dihydroxyvitamin D3 Glykosiden bei milchgebenden Tieren
Utilisation d'1,25-dihydroxyvitamine D3 glycosides chez des animaux producteurs de lait

(30) Priority: 10.06.2009 BE 200900354; 14.09.2009 EP 09170222
(43) Date of publication of application: 18.04.2012
(73) Proprietor: Emma ® Nutrition, 8970 Poperinge (BE)
(72) Inventor: VERSCHAEVE, Maurice, B-8970 Poperinge (BE)
(74) Representative: Caers, Raphael Frans Ivo
(86) International application number: PCT/IB2010/052467
(87) International publication number: WO 2010/143101

(56) References cited:
- EP-A1- 0 482 947
- WO-A1-2009/129818
- AT-B- 398 372
- US-A- 5 869 083
- HORST RL ET AL: "Using Solanum Glaucophyllum as a Source of 1,25-dihydroxyvitamin D to Prevent Hypocalcemia in Dairy Cows" ACTA VETERINARIA SCANDINAVICA, BIOMED CENTRAL LTD, LO, vol. 44, no. Suppl 1, 31 March 2003 (2003-03-31), page P67, XP021026562 ISSN: 1751-0147 cited in the application
- KUNZ W ET AL: "[Solanum malacoxylon: studies of tolerance of doses recommended for the prophylaxis of parturient paresis]" BERLINER UND MÜNCHENER TIERÄRZTLICHE WOCHENSCHRIFT 1 NOV 1981, vol. 94, no. 21, 1 November 1981 (1981-11-01), pages 421-424, XP008116775 ISSN: 0005-9366
- ROUX R ET AL: "SOLANUM-GLAUCOPHYLLUM IN PREGNANT COWS EFFECT ON COLOSTRUM MINERAL COMPOSITION AND PLASMA CALCIUM AND PHOSPHORUS LEVELS IN DAMS AND NEW BORN CALVES" ANNALES DE BIOLOGIE ANIMALE BIOCHIMIE BIOPHYSIQUE, vol. 19, no. 1A, 1979, pages 91-102, XP008116789 ISSN: 0003-388X
- M. Helena Amaral et al.: "Effect of Hydroxypropyl Methylcellulose and Hydrogenated Castor Oil on Naproxen Release From Sustained-Release Tablets" AAPS PharmSciTech vol. 2, no. 2, 6, 2001, 2001, pages 1-8, XP002562474 Retrieved from the Internet: URL:http://www.aapspharmscitech.org/view.a sp?art=pt020206 [retrieved on 2010-01-05]

## Description

Hypocalcaemia is a metabolic disease which mainly occurs with lactating animals such as cows, dairy cows, sheep, goats, but also with horses. The disease mainly occurs in older highly productive animals which have already calved multiple times. Although hypocalcaemia appears primarily as subclinical hypocalcaemia in the early postpartum phase and in the lactation period several weeks after calving, the period in which it manifests itself may vary from a few weeks before up to a few weeks after calving. The lactation cycle of a dairy cow starts immediately after calving and extends over approximately 10 months of the year.

First symptoms of hypocalcaemia are the loss of mental arousal and reduced appetite. Changes may occur within a relatively short time span of a couple of minutes, up to a longer time span of several hours. A quick treatment of the animal is advised in order to improve the response. Serious cases of hypocalcaemia are known as causing birth paresis or milk fever which may evolve to paralysis predominantly at the rear side of the animal, and can lead to the death of the animal.

The occurrence of hypocalcaemia is attributed to an excessively low calcium level in the blood, which mainly occurs around the period of calving. In this period the amount of Ca necessary for the production of colostrum is much higher than the amount available in the blood, obtained by intake through the bowels and through mobilization from the bones. During the dry period, i.e. a period of approximately two months preceding the calving in which the animal is not milked, the lactation is minimal and the Ca-need is significantly lower. The cow is then capable of resorbing a sufficient amount of calcium from the bowel. During this dry period, the calcium need for maintaining the metabolism and the growth of the foetus is limited (< 30 g) while the quantity of calcium in normal food is substantially higher. As a result of this, the body of the animal reacts by decreasing the gastrointestinal absorption of calcium. The excretion of hormones responsible for an active calcium transport originating from the gastrointestinal tract and for the mobilization from the bones to the blood (parathyroid hormone, 1,25-Dihydroxy-Vitamin D) is therefore at a minimum during this period. Around the time of calving lactation is induced and the calcium need increases substantially. Milk production subsequently goes through a peak at 4-6 weeks after calving. Large amounts of calcium are then transported from the blood to the lacteal gland, as a result of which the quantity of calcium in the blood decreases significantly. If the concentration decreases below 1.8 mMol/l, some cows may show symptoms of hypocalcaemia. The beginning of lactation puts severe pressure on the Ca-balance and despite hormonal regulation, which cause the activation of vitamin D dependent mechanisms, a Ca-deficiency (hypocalcaemia) occurs to some degree with every cow. Hypocalcaemia usually occurs within 72 hours after calving. Most cows possess a natural resistance mechanism which enables them to provide a sufficient calcium supply, independent of the calcium originating from nutrition, through intake from the bowel and resorption from the skeleton. However, in 5-20% of all cows the recovery of the Ca equilibrium is not achieved through intervention of the parathyroid hormone and they develop milk fever or hypocalcaemia.

With cows suffering from hypocalcaemia, generally three stages of the disease are recognized.

Disease stage 1: No typical symptoms. Observed signs which only attract attention after observation during approximately 1 hour: lack of appetite, excitability, nervousness, weakness, weight shifting and shuffling of the back feet, the cow exhibits poor coordination of its movements, muscle trembling and quivering, cooling of the large muscle groups of the animal, particularly a cold rear end of the animal.

Disease stage 2: Lasting 1 to 12 h. The cows are unable to stand but can maintain sternal recumbancy, the head usually turned towards the body. The affected animals appear dull and listless. The digestive tract is inactive, heart rate increases above 100 bpm and the body temperature reduces.

Disease stage 3: Progressive loss of consciousness (finally coma) is observed. Further increase of the heart beat rate which may approach 120 bpm and the body temperature further decreases. Untreated cows may die within 12h of onset of the first clinical signs.

Several methods have been developed for the treatment of hypocalcaemia. Examples are amongst others intravenous (IV) administration of calcium which gives a short term recovery; IV administration of a Ca/Mg infuse the day after calving; the injection of a synthetic vitamin D preparation in order to stimulate the Ca-intake in the bowel, which is forbidden in many countries despite its good results; the administration through the mouth of vitamin D2 or the administration through an injection of vitamin D3 or dihydrotachysterole in the muscles. The disadvantage is that these compounds still have to be metabolized and that they are not stable in an organic environment. Also, only a small part of the administered compounds is converted into the active metabolite 1,25-dihydroxyvitamin D3, as a result of which large quantities have to be administered in order to build a therapeutic concentration of the active metabolite. Another method aims at controlling the anion-cation balance in the feed ration during the last few weeks preceding the calving. High anionic feed rations which predominantly comprise chloride and sulfur, seem to be able to reduce the percentage of milk fever occurrence, because they reduce the pH of the blood and thus increase the intake of Ca from the bones and from the food.

EP1162890 describes a method for the prevention of hypocalcaemia in a lactating animal, whereby during at least a part of the dry period, food is supplied which contains an effective amount of a compound, which reduces the absorption of calcium from drinking water and/or from the feed ration of the animal by binding the free calcium present in the gastro-enteritic channel. Because the free calcium can not be absorbed by the animal, the natural calcium regulating defense mechanism is stimulated. Examples of suitable calcium binding compounds are oxalic acid, sodium oxalate, fytinic acid, a fytate, mineral clay, zeolites, and others. This method however demands a strict organization and a proper feeding of the cows being in their dry period.

Gerloff, et al., "Dry Cow Feeding and Metabolic Problems", 46th Minnesota Nutritional conference and Monsanto Technical Symposium, September 16-18, 1985 suggest to avoid an excessive intake of calcium and phosphor in the period preceding calving to prevent milk fever. Jorgensen, N. A., "Combating Milk Fever", Journal of Dairy Science, Vol. 57, No. 8, 1973, pp. 933-944 gives an overview of known methods for the treatment of milk fever. As examples are mentioned prepartal diets with a low calcium level which regulate the calcium/phosphor ratio, the administration of acidic foods, the prepartum administration of mineral acids or ammonium chloride, daily administration of 90 to 100 g of calcium chloride during a short period, the prepartum administration of large doses of vitamin D or 25-hydroxycholecalciferol. Howard, Ed., "Current Veterinary Therapy I: Food Animal Practice", W. B. Saunders Co., Philadelphia, 1981, pp. 340-343 describes the antepartum administration of vitamin D2 and a vitamin D3 metabolite antepartum to reduce the occurrence of milk fever. Also the oral administration of 90 to 100 g of calcium chloride during two or three days antepartum is described.

From US-A-4.931.290 it is known to reduce the risk for the occurrence of milk fever after calving by administering to the animal after calving, every 8-16 hours, an oral dose of an effective amount of (1) a water soluble calcium salt, (2) an effective amount of a complexing agent for complexating phosphorus into a non-water-soluble phosphorus complex, and (3) approximately 17 mg of magnesium or an equivalent metal. Administration of Ca and binding of phosphorus in an insoluble complex have the effect that the serum calcium concentrations are kept at a level which prevents serious forms of milk fever. The administered dose may also comprise vitamin D3. The dose is administered in the form of a powder, tablet, capsule or bolus. It is suggested to administer the first three doses during the first 30 hours after calving, such as immediately after calving and after 12 and 24 hours, typically followed by additional doses 36, 48, and 72 hours after calving. The repeated administration after calving however negatively influences the comfort and well-being of the animal and demands an intensive control by the cattle breeder. An additional disadvantage is that the vitamin D3 is insufficiently metabolized.

US2006/270640 describes the transvaginal administration of 1[alpha]-hydroxyvitamin D3 or 1,25-dihydroxyvitamin D3 for the treatment of hypocalcaemia, more specifically for the treatment of disorders such as intrapartum astasia, which is an important cause of death in cows. Vitamin D, A, and E are rather fat-soluble, and only a special vitamin D derivate mav be absorbed from the vaaina in a sufficient quantity to prevent milk fever. This method is however uncomfortable for the animal.

WO 2009/129818 A1 discloses the use of an enriched extract of Solanum glaucophyllum with 1,25-dihydroxyvitamin D3 glycosides or their synthetic analogs, together with flavonols, particularly quercetin glycosides, for the treatment or prevention of hypocalcemic paresis around parturition in milk producing animals and any diseases related to a decline in plasma calcium levels during calving in cattle and other milk producing animals. The administration may be oral, e.g. in capsules or tablets. Retard formulations are also possible, but the document does not disclose how any retard release should be obtained, in particular not with oral administration to ruminants. Inorganic matter may also be present in the formulation as inactive components. The administration occurs with pregnant cows in a single and immediate dose of 5g between 72-24 hrs before calving. WO 2009/129818 A1 is however silent about the exact amounts of the active substances which were administered as part of that dose. A disadvantage of the disclosure in WO 2009/129818 A1 is that first an enriched extract of Solanum glaucophyllum needs to be made, which adds additional complexity to the preparation of the composition which is to be administered. Another disadvantage is that the active substances are administered as an immediate dose, of which the effects may reduce rather quickly over time, and which represents a particular problem if exact timing of the administration to pregnant cows, in view of the difficulty in predicting the exact moment of calving. A further disadvantage is that the composition proposed in WO 2009/129818 A1 is a synthetic drug, which needs to pass the various official drug registration procedures, which are time consuming, costly and complex, before it may be commercialised. WO 2009/129818 A1 is thus concerned with the production and administration of a synthetic drug, and therefore belongs to a different field from this of the present invention.

In "Using Solanum glaucophyllum as a source of 1,25-dihydroxyvitamin D to prevent hypocalcemia in dairy cows", Horst et al., Acta vet; scand. Suppl. 98-2003, hypocalcaemia is fought with gelatin boluses comprising leafs of Solanum glaucophyllum (Sg). The gelatin boluses were administered to cows which already received a diet having a reduced cationanion balance. Boluses made of gelatin have the characteristic of dissolving relatively fast in the stomach, and of releasing their active ingredients usually within a period of 12 hours. Solanum glaucophyllum (Sg) is a plant comprising high quantities of 1,25-dihydroxyvitamin D3 glycoside. In this publication, several experiments with gelatin boluses are described. The final conclusion is that best results are obtained by a phased administration. Hereby from 5 days prepartum until 7 days postpartum, a bolus comprising 2g Sg was administered daily, from day 8 to day 14, a bolus comprising 1g Sg was administered daily, and from day 15-21 the daily bolus comprised 0.5 g. Although good results were obtained with this method, the method is uncomfortable for the animal; given that it gets a bolus administered. every day, and for the person taking care of the animals, given that the daily administration of the boluses is a labor intensive activity, and moreover the prepartum date is difficult to predict accurately.

Although diverse remedies and methods have been developed for the prevention and the treatment of hypocalcaemia, there is still a need for a remedy which is capable of overcoming hypocalcaemia in an efficient way, both in the early phase where hypocalcaemia manifests itself as reduced appetite, as in the later phase where signs of paralysis may occur. Moreover, there is a need to provide as much comfort as possible to the animal and to the person taking care of the animal.

The object of this invention is therefore to prevent a symptom in a lactating animal, chosen from reduced appetite, paralysis or a combination of both, in the period after calving or giving birth to young animals, in a way that provides optimum comfort to the animal and to the person taking care of the animal. The prevention or avoidance of these effects is of significant importance tor dairy cattle breeders, both in terms of work load as in emotional strain, as well as commercially, since they strongly influence the milk production of the animals.

This is achieved according to the present invention by the composition for use as defined by claim 1.

Preferably the leafs originate from Solanum glaucophyllum. The inventor has namely found that 1,25-dihydroxyvitamin D3 glycoside is taken up by the blood almost immediately; also when administered orally. Glycoside is metabolically separated in the gastro-enteritic system, after which the 1,25-dihydroxyvitamin D3 is biologically available in the animal to provide an increased intestinal calcium uptake by the blood. The biological effect of 1,25-dihydroxyvitamin D3 glycoside starts within a short time after intake and ensures that the natural defense mechanism of the animal is stimulated shortly after intake. 1,25-dihydroxyvitamin D3 glycoside is metabolized by cleavage of the glycoside and directly moves to the bowel where it can be taken up into the blood, in contrast to vitamin D3 as such. By administering the composition prior to lactation, at a point in time where the animal still has a good appetite and the Ca deficiency hasn't occurred yet, the animal has the capability to build a good resistance, so that the risk for the occurrence of a reduced appetite and signs of paralysis associated with hypocalcaemia after giving birth may be minimized in an early stage. The delayed release of the 1,25-dihydroxyvitamin D3 glycoside ensures that the optimum gastro-intestinal Ca intake is spread over a longer period. This is important given the fact that loss of appetite and signs of paralysis may also occur in a somewhat later stage, i.e. a few days up to a few weeks after calving. The composition for use according to this invention may be administered perorally in any manner desired, but is preferably administered with a shooting mechanism through the mouth to ensure direct administration into the stomach.

The composition for use according to this invention preferably has a density of at least approximately 1.25 kg/l as a result of which it sinks, after intake in the stomach, directly to the bottom of the stomach, more specifically to the bottom of the second stomach or the first stomach, where it remains, at minimal risk of being mixed with the food which is being ruminated. This way an optimum delivery of 1,25-dihydroxyvitamin D3 glycoside to the bowel is guaranteed. Preferably however the composition has a density which is at least 1.5 kg/l, more preferably 1.75 kg/l. Such a high density may for example be obtained by adding one or more metals or metallic compounds to the composition. Examples of suitable metals are iron, iron oxides or other iron compounds. These show minimal risk for intoxication. Also other metals or metallic compounds may be used, such as zinc powder, cupper powder, but their concentration has to be chosen in such a manner that intoxication is prevented. Preferably approximately 20-60 wt.% of metal powder is added to the composition for use according to the present invention, more preferably approximately 25-55 wt.% of metal powder, more preferably approximately 30-50 wt.% of metal powder, most preferably approximately 40 wt.% of metal powder. Preferably the metal powder is iron powder.

Because the active compound is released from the composition in a delayed manner, one single dose may be sufficient, provided the dosing of the 1,25-dihydroxyvitamin D3 glycoside is adequately chosen. Thanks to the delayed release which is achieved because the material of the bolus slowly decomposes or physically erodes, a similar effect is obtained as in a phased administration of leafs which comprise 1,25-dihydroxyvitamin D3 glycoside, but this time with only one single administration. The fact that one single peroral administration suffices, increases the comfort of the animal and the person taking care of the animal considerably. The delayed release is not only achieved by the material of the bolus which slowly erodes, but also because the 1,25-dihydroxyvitamin D3 is bound to a glycoside structure, which has to be cleaved enzymatically in the cow's metabolism and because the 1,25-dihydroxyvitamin D3 first has to be released from the leafs.

The cleaving of the glycoside group in the rumen flora for example takes approximately 8-12 hours. As a result of this, the 1,25-dihydroxyvitamin D3 is released. This 1,25-dihydroxyvitamin D3 then becomes available for uptake within 15 min - 6 hours. Because of this cleavage of the glycoside, the delivery of 1,25-dihydroxyvitamin D3 is further delayed.

Because of the fact that the active compound 1,25-dihydroxyvitamin D3 glycoside is still encapsulated in the leafs, yet a further delayed release of the active substance is achieved, which is a further advantage for the action of the composition for a single administration.

Finally a composition with a stable delayed release is hereby obtained which lasts approximately 10-12 days, whereby the delivery decreases towards the end. Because of this decrease towards the end, habituation to the product is avoided, as a result of which there is usually no fallback-effect upon an ending of the treatment, and as such, the physiological condition of the animal is kept optimal. Such a delayed release with a decrease towards the end, is at the moment not known for a treatment for hypocalcaemia with a single administration, and thus offers considerable advantages both for the animal and for the person taking care of the animal. By the single administration and the delayed action, the milk production after calving, as well as a good condition of the animal, is assured. The parathyroid gland can also slowly start to work and regain its production of the parathyroid hormone, as a result of which the full biological process runs normally.

Where known techniques only focus on increasing the Ca concentration in the blood or on stimulating the Ca production during the dry period, and thus intervene in the Ca/Mg metabolism of the animal which can cause undesired side effects, this invention focuses on enabling a more sufficient utilization of the abundant free Ca present in the bowel through the food, without substantially intervening in the Ca/Mg metabolism. This is an important advantage because changing Ca/Mg concentrations may give rise to among others the occurrence of cramps.

The 1,25-dihydroxyvitamin D3 glycoside used in the composition for use according to this invention is preferably derived from the plant Solanum glaucophyllum. It is preferably obtained by preparation of the leafs of Solanum glaucophyllum, more specifically by standardizing the content of 1,25-dihydroxyvitamin D3 glycoside originating from Solanum glaucophyllum. Hereby large batches of Solanum glaucophyllum are thoroughly ground and mixed, after which the correct average concentration of 1,25-dihydroxyvitamin D3 glycoside is determined, which applies to the entire batch because of the thorough mixing. After this, small quantities of these ground leafs can be weighed to obtain with a considerably high precision a certain specific quantity of 1,25-dihydroxyvitamin D3 glycoside per dose (or bolus). In this manner, a constant and accurate quantity of 1,25-dihydroxyvitamin D3 glycoside may be assured in the bolus. This Solanum glaucophyllum mixture of leaf parts comprising 1,25-dihydroxyvitamin D3 glycoside is commercially available as a composition which usually comprises approximately 20 ppm of 1,25-dihydroxyvitamin D3 glycoside (20 mg per kg).

To facilitate the administration and thus to keep the comfort of the animal as high as possible, the composition is administered in the form of a, tablet or bolus, whereby the use of the bolus is preferred.

In case the composition is administered as a tablet or bolus, this is administered preferably through the mouth using a shooting device so that it is ensured that the composition will end up in the second stomach or the first stomach, and the risk for rumination is kept minimal. Due to its high density, the bolus will stay at the bottom of the second stomach or first stomach with a very low risk to being ruminated and the 1,25-dihydroxyvitamin D3 glycoside is released from the second stomach or the first stomach in a delayed process. The rumen is the first stomach, with a capacity of approximately 200 liters. Typically the bolus will stay in this stomach, although the bolus may in certain cases also move to the second stomach or reticulum. Whether the bolus stays in the reticulum or the rumen, does practically not make much of a difference for its action. Because of the physiological structure of the stomachs in ruminant animals such as a cow, the chance that the bolus will move to a third stomach is very unlikely. If the bolus would be regurgitated and ruminated by the animal, this still is not problematic since the cow ruminates the food into small pieces and these pieces would land on the bottom of the reticulum or the rumen where they further disintegrate.

The inventor has found that due to its high density, the bolus stays after intake on the bottom of the second stomach or the first stomach, with a very low risk for being regurgitated during rumination and for the 1,25-dihydroxyvitamin D3 glycoside being released from the leafs much faster than intended. The weight and dimensions of the bolus may be varied within large ranges, but they are preferably adjusted to the weight of the animal, so that only one administration suffices.

The dose of 1,25-dihydroxyvitamin D3 glycoside administered to the animal is preferably at least 460 µg, preferably 550 µg. This dose is chosen in such a way that the amount of 1,25-dihydroxyvitamin D3 glycoside administered is sufficient to achieve the preventive effect. This dose is chosen in such a way that the amount of administered 1,25-dihydroxyvitamin D3 glycoside is sufficient to achieve that the amount of Ca, which is resorbed daily from the gastro-enteritic channel, comprises at least a part of the normal daily amount, preferably at least the normal daily amount. A dose of 450 to 500 µg divided over minimum 10 days results in an average delivery of maximum 50 µg per day. If extracts of Solanum glaucophyllum are used instead of the leafs, or if synthetic 1,25-dihydroxyvitamin D3 is administered, the delivery is delayed less, which is disadvantageous. There is also no biological cleaving of the glycoside. If there is no delayed release, or a less delayed release, the window for the therapeutic administration in a single administration becomes very small. The active compound will be released a lot faster than with the delayed release. Consequently the bolus will have to be administered at a certain exact moment prior to calving which has to be determined precisely. If this does not happen, one runs the risk of releasing the active compound too soon or too late. Given that the time of calving is however difficult to predict, there is a relatively high chance that such a bolus will not be administered at the right moment. In this case or in case accidentally anything goes wrong in the delayed release of the active compound from the composition and a larger amount of the 1,25-dihydroxyvitamin D3 glycoside is released in one time, there are no direct negative consequences for the animal, but the protective effect will possible disappear partially or completely.

Delayed release of 1,25-dihydroxyvitamin D3 glycoside is achieved by encapsulating the 1,25-dihydroxyvitamin D3 glycoside in a material or a compound which is slowly decomposed or physically eroded in the stomach. The compounds for this purpose are chosen from the group of a fat, for example castor oil which may be completely or partially hardened by hydrogenation, or a fat derivate, but also a polysaccharide, a cellulose, a gum, a glycol and derivates of these compounds can suitably be used. Castor oil which has been hydrogenated and very strongly compressed usually works for 10-12 days. It is important that the rate.of hydrolysis of the compound is sufficiently low, to be able to assure a delayed release of the active compound. It is also important that the melting point of the substance is quite high, preferably around are above 88°C. This is also important in the production of the composition, given that the compound has to be mixed in a molten condition with the leafs and the other ingredients. A preferred embodiment of such a process is described hereunder. Preferably fully hydrogenated castor oil is used. This compound is very suitable for producing a composition for a delayed release of 1,25-dihydroxyvitamin D3 glycoside, given the optimal rate at which the material physically erodes. This compound can also be compressed very well in combination with for example iron powder, which is used to make the composition heavier. In addition to the castor oil, magnesium stearate is preferably also added, as a lubricant during the compression of the composition. If so desired the composition with the 1,25-dihydroxyvitamin D3 glycoside may be encapsulated in a membrane, for example a membrane of the previously mentioned compounds or a foil of a plastic material which is biologically degradable in the stomach, such as a polyester, for example polylactic acid. The compounds which control the delayed release of the 1,25-dihydroxyvitamin D3 glycoside, are chosen such that the release continues during a period of at least 3 days, preferably at least 5 days. Release will usually stop within a period of 20 days after administration, preferably within a period of at least 10 days.

Preferably the size and shape of the bolus are also determined in function of the material or the compound chosen for the bolus, so that the periods of release of the active compound, as described here above, are obtained. In a preferred embodiment of the bolus in which hardened or hydrogenated castor oil with a little bit of magnesium stearate is used as a slowly eroding material, the bolus may for example have a length of 80 mm with a thickness and a width of 25 mm, preferably with rounded shapes and/or corners to decrease the risk for injuries.

The composition may further also comprise 1,25-dihydroxyvitamin D3 glycoside metabolites.

The composition for use according to this invention is meant for peroral administration, preferably at a time shortly before calving but sufficiently long before calving to guarantee an optimum Ca intake for as long as an increased Ca need exists. Preferably the composition or bolus is administered at least 1 day prior to calving, preferably 1 or 2 days prior to calving.

In a useful embodiment the composition for use according to this invention comprises additives which may support or improve the effect of the composition, such as for example other vitamins or derivates thereof, such as vitamin A, B12, C, D, E, K, niacin, thiamin, choline, biotin, folium acid, riboflavin, pantothenic acid, one or more minerals, chosen from the group of cobalt, cupper, manganese, selenium, zinc, or a carrier for the 1,25-dihydroxyvitamin D3 or the glycoside. Use of a carrier may be useful for improving the dispersability into or between the other components of the composition. Examples of suitable carrier materials are food fibers, wrack.

The composition for use according to this invention is suitable for use in lactating animals, for example cows, sheep, goats, horses, donkeys, etc.

This disclosure also relates to the use of 1,25-dihydroxyvitamin D3 glycoside for the preparation of a composition with a density of at least 1.25 kg/l which causes a delayed release of 1,25-dihydroxyvitamin D3 glycoside, with the purpose of increasing intestinal calcium intake by blood of an animal around and after calving.

Another aspect of this disclosure relates to the use of a 1,25-dihydroxyvitamin D3 glycoside comprising composition as described here above to prevent a symptom chosen from reduced appetite, paralysis or a combination of both in an animal after calving.

Still another aspect of this disclosure relates to the use of a 1,25-dihydroxyvitamin D3 glycoside comprising composition as described here above, for increasing intestinal calcium intake by blood of an animal after calving.

The invention further relates to a composition as defined in claim 9.

The fat derivate is preferably castor oil, preferably at least partially hydrogenated castor oil, but preferably highly to fully hydrogenated or hardened castor oil.

Useful compositions have the form of a tablet, or a bolus. Useful compositions may also have the form of a bolus taken up in a packaging which is preferably porous to on one hand enable diffusion of the gastric juice into the content of the sack for dissolving 1,25-dihydroxyvitamin D3, and on the other hand to enable outward diffusion of the dissolved 1,25-dihydroxyvitamin D3 glycoside.

The composition may further comprise other useful ingredients such as at least one other vitamin or mineral, for example vitamin A, B12, C, D, E, K, niacin, thiamin, choline, biotin, folium acid, riboflavin, pantothenic acid, one or more minerals, chosen from the group of cobalt, cupper, manganese, selenium, zinc, or a carrier for the 1,25-dihydroxyvitamin D3 glycoside.

The invention is further illustrated by means of the following examples.

The natural glycoside form of 1.25-dihydroxyvitamin D3 as it is used in preferred embodiments offers a unique stability and water solvable characteristics for an increased activity. This form is also heat resistant up to 100 °C.

This preferred embodiment of a bolus allows the administration of one single treatment, shortly prior to calving. A sustained release of the active ingredient during a period of 10-12 days corresponds with a phased administration, as this is described in the prior art. In the prior art, several gelatine boluses however have to be administered, which release the active ingredient during a period of approximately 12 hours, while the present invention achieves the same effect with one single bolus.

### EXAMPLE

Before producing the bolus, the leafs of Solanum glaucophyllum are ground and standardized. This means that the leafs are ground and mixed in large quantities to obtain a single uniform batch. Subsequently, the average concentration of the 1,25-dihydroxyvitamin D3 present in the leafs is determined as accurately as possible. Based on this concentration, one doses the 1,25-dihydroxyvitamin D3 into individual portions, by weighing a well determined quantity of ground leafs and mixing them again. Given the intensive mixing and grinding of the batch of leafs, one may assume that the concentration of 1,25-dihydroxyvitamin D3 through the mixture is fairly constant, as a result of which the weighed doses of the leafs will all comprise approximately the same and predetermined quantity of 1,25-dihydroxyvitamin D3.

In a porous nylon sack having pores of 37 µm, a bolus was placed with a weight of 70.2 g, which was prepared from amongst other things 18.4 g of hydrogenated castor oil (obtained under the tradename Cutina® HR PH from COGNIS and 23.3 g of Panbonis® delivered by HERBONIS. the latter was a composition of ground and standardized Solanum glaucophyllum leafs, which comprised approximately 20 ppm of 1,25-dihydroxyvitamin D3 glycoside. This bolus corresponded with an average daily release of 38-46 µg of the active ingredient 1,25-dihydroxyvitamin D3 glycoside over 10-12 days. The bolus further comprised 27.6 g of iron powder and 0.9 g of magnesium stearate. The iron powder only has a ballast function and served to make the bolus,heavier so that it will stay on the bottom of the stomach of the animal. The magnesium stearate is a component which together with the castor oil serves as a lubricant enable compression of the obtained powder.

During production of the bolus itself, the iron powder was mixed with the Solanum glaucophyllum leafs and heated to 85°C. The hydrogenated castor oil (E498) was also heated to above its melting point of 88°C. E498 is the E-number of castor oil. With E-numbers a list of additives is meant, which are allowed by the European Union for use in food products for human consumption. Subsequently these products were mixed together at this high temperature. After thorough mixing, the mixture was cooled down to 15°C. The mixture of Solanum glaucophyllum leafs, iron powder and castor oil was cooled down to 15°C, after which it was ground very fine into the form of a powder. After this the magnesium stearate (E571) was added and mixed with the rest.

The final mixture was pressed to form a bolus in a compression machine with a mold shaped like a bolus. The composition of the bolus had a density of 1.75 kg/l. The composition was pressed to a substantially cylinder-shaped bolus having a length of 80 mm, a thickness of 25 mm and a width of 25 mm with rounded ends.

An analysis of the composition of the bolus provided the results in weight units given in table 1.

**Table 1.**

| | |
|---|---|
| water | 4,30% |
| raw protein | 6,60% |
| raw fat | 21,20% |
| raw fibre | 8,70% |
| raw ash | 53,66% |
| total carbohydrates | 5,53% |

A sack with a bolus was hung in the first stomach of 15 cows which had already calved at least 3 times, and thus had an increased risk for the occurrence of hypocalcaemia, loss of appetite and signs of paralysis. Every day at the same time the sack was removed from the first stomach, rinsed with water and weighed after draining.

During the first 4 days it was noticed that the weight of the bolus increased by absorption of first stomach fluid. After this period the weight decreased again. The bolus was nearly completely resorbed after 14 days. The evolution of the weight of the bolus in function of time is shown in the following table.

With none of the cows a loss of appetite was determined, and also no signs of paralysis occurred. The Ca blood concentration in the period preceding calving and at least 2 days after calving remained stable.

The effect on the blood parameters was measured in 9 cows which had previously calved 3 or more times. In table 2, the calcium content in the blood of the cows is shown for a first blood sample taken upon application of the bolus 1-2 days prior to calving and of a second blood sample taken 24 hours after calving. Cows 1 and 2 did not receive a bolus. As an optimum calcium content for the blood plasma of dairy cows, 2.2 mmol/l Ca is accepted.

**Table 2.**

| Cow | 1-2 days prior to calving (mmol/l Ca) | 24 hours after calving (mmol/l Ca) |
|---|---|---|
| nr. 1 | 2,33 | 2,13 |
| nr. 2 | 1,93 | 1,83 |
| nr. 3 | 1,87 | 2,47 |
| nr. 4 | 1,98 | 2,16 |
| nr. 5 | 1,96 | 2,16 |
| nr.6 | 2,51 | 2,16 |
| nr. 7 | 2,1 | 2,21 |
| nr. 8 | 2,2 | 2,25 |
| nr.9 | 2,24 | 2,33 |

Cow 2 was treated after taking the second sample because disease symptoms such as a beginning paralysis were observed. In this cow, a Ca-content of 2.93 mmol/l was measured in the blood 24 hours after this treatment.

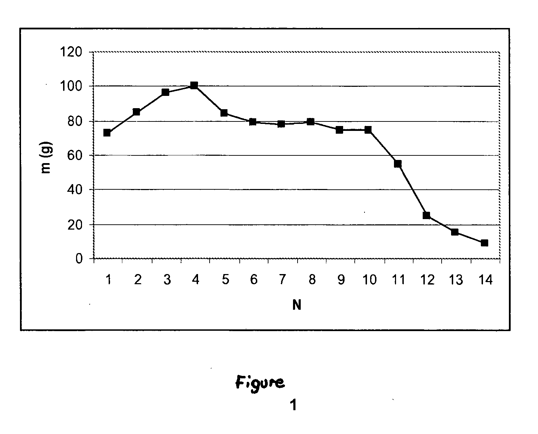

In table 3, the kinetics of the bolus are shown. Hereby the weight of the bolus is shown in function of the number of days the bolus was present in the rumen. Figure 1 graphically shows the progress of the weight of the bolus (m) expressed in grams in function of the number of days (N) the bolus had been present in the rumen.

### Bolus kinetics and disintegration in the rumen:

**Table 3**

| **Days in first stomach (N)** | **Weight of bolus (m) (grams)** |
|---|---|
| 1 | 72.6 |
| 2 | 85.0 |
| 3 | 96.2 |
| 4 | 100.0 |
| 5 | 84.4 |
| 6 | 79.3 |
| 7 | 77.7 |
| 8 | 79.0 |
| 9 | 75.0 |
| 10 | 74.5 |
| 11 | 55.2 |
| 12 | 24.7 |
| 13 | 15.5 |
| 14 | 8.7 |

From these experiments, it is clearly visible that the bolus according to the present invention increases the calcium concentration in the blood, as a result of which the frequency of the occurrence of hypocalcaemia will be decreased.

## Claims

1. A composition containing leafs originating from plants comprising 1,25-dihydroxyvitamin D3 glycoside, the composition being for a single peroral administration in the form of a tablet or a bolus, the composition comprising:
- a compound or a material which slowly decomposes or physically erodes in the stomach, in which compound or material the leafs are encapsulated, as a result of which a delayed release of 1,25-dihydroxyvitamin D3 glycoside is realized, the compound or material being chosen from the group consisting of a fat or a derivative thereof, a polysaccharide, a cellulose, a gum, a glycol and derivates of these compounds useful for the delayed release of the 1,25-dihydroxyvitamin D3 glycoside, whereby the compounds which control the release of the 1,25-dihydroxyvitamin D3 glycoside are chosen such that the release continues during a period of at least 3 days, and
- one or more metals or metallic compounds so that the composition obtains a weight density which causes it to sink, after administration, to the bottom of the stomach, and to remain there,
for use in the prevention of a symptom chosen from reduced appetite, paralysis or a combination of both, with an animal after having given birth or after calving, whereby the composition is to be administered perorally as a single administration.

2. The composition for use according to claim 1 wherein the leafs originate from Solanum glaucophyllum.

3. The composition for use according to claim 1 or 2 wherein the composition has a density of at least 1.5 kg/l.

4. The composition for use according to any one of claims 1-3, whereby in the composition for single administration at least a quantity of leafs comprising 0.460 mg of 1,25-dihydroxyvitamin D3 glycoside is administered to the animal as a single dose.

5. The composition for use according to claim 4, wherein the quantity of leafs comprises 0.550 mg of 1,25-dihydroxyvitamin D3 glycoside.

6. The composition for use according to any one of claims 1 to 5, wherein a fat derivative is chosen and wherein the fat derivative is an at least partially hardened or hydrogenated castor oil.

7. The composition for use according to any one of claims 1-6, whereby the composition is provided for administration at least 1 day preceding calving, preferably 1 or 2 days preceding calving.

8. The composition for use according to any one of claims 1-7, wherein the composition further comprises one or more components chosen from the group of vitamin A, B12, C, E, K, niacin, thiamin, choline, biotin, folium acid, riboflavin, pantothenic acid, one or more minerals, chosen from the group of cobalt, cupper, manganese, selenium, zinc.

9. A composition for preventing by administration as a single peroral administration a symptom selected from reduced appetite or signs of paralysis or a combination thereof associated with giving birth by an animal which, in a form suitable for peroral administration in the form of a tablet or bolus, comprises leafs of plants with, in those leafs, at least 0,460 mg of 1,25-dihydroxyvitamin D3 glycoside as well as a compound or a material which slowly decomposes or physically erodes in the stomach, in which compound or material the leafs are encapsulated, the compound or material being chosen from the group consisting of a fat or a derivative thereof, a polysaccharide, a cellulose, a gum, a glycol and derivates of these compounds useful for the delayed release of the 1,25-dihydroxyvitamin D3 glycoside, whereby the compounds which control the release of the 1,25-dihydroxyvitamin D3 glycoside are chosen such that the release continues during a period of at least 3 days, and one or more metals or metallic compounds so that the composition obtains a weight density which causes it to sink after administration to the bottom of the stomach, and to remain there.

10. The composition according to claim 9 wherein the leafs contain at least 0,550 mg of 1,25-dihydroxyvitamin D3 glycoside.

11. The composition according to claim 9 or 10, wherein a fat derivative is chosen and wherein the fat derivate is an at least partially hardened or hydrogenated castor oil.

12. The composition according to any one of claims 9-11, wherein the composition is in the form of a bolus and is taken up in a packaging which is porous.

13. The composition according to any one of claims 9-12 in which the composition has the form of a bolus.

14. The composition according to one of claims 9-13, wherein the composition further comprises at least one vitamin or mineral.

## Patentansprüche

1. Zusammensetzung enthaltend Blätter, die von Pflanzen stammen, umfassend 1,25-Dihydroxyvitamin-D3-Glycosid, wobei die Zusammensetzung für eine einmalige perorale Verabreichung in Form einer Tablette oder eines Bolus dient, wobei die Zusammensetzung Folgendes umfasst:
- eine Verbindung oder ein Material, die/das im Magen sich langsam abbaut oder körperlich erodiert wird, in welche Verbindung oder in welches Material die Blätter eingekapselt sind, wodurch eine verzögerte Freisetzung von 1,25-Dihydroxyvitamin-D3-Glycosid erreicht wird, wobei die Verbindung oder das Material aus der Gruppe ausgewählt werden bestehend aus einem Fett oder einem Derivat davon, einem Polysaccharid, einer Cellulose, einem Gummi, einem Glycol und Derivaten dieser Verbindungen, die für die verzögerte Freisetzung des 1,25-Dihydroxyvitamin-D3-Glycosids nützlich sind, wobei die Verbindungen, die die Freisetzung des 1,25-Dihydroxyvitamin-D3-Glycosids regulieren, so ausgewählt werden, dass die Freisetzung während einer Zeitspanne von mindestens 3 Tagen fortdauert, und
- ein oder mehrere Metalle oder metallische Verbindungen, so dass die Zusammensetzung eine Masse/Volumen-Dichte erhält, die sie nach Verabreichung zum Absinken auf den Magenboden und dort Verbleiben bringt,
zur Verwendung zur Verhinderung eines Symptoms ausgewählt unter mangelndem Appetit, Lähmung oder einer Kombination von beiden bei einem Tier, nachdem es gejungt hat oder nach dem Kalben, wobei die Zusammensetzung peroral als einmalige Verabreichung verabreicht werden soll.

2. Zusammensetzung zur Verwendung nach Anspruch 1, wobei die Blätter von Solanum glaucophyllum stammen.

3. Zusammensetzung zur Verwendung nach Anspruch 1 oder 2, wobei die Zusammensetzung eine Dichte von mindestens 1,5 kg/l aufweist.

4. Zusammensetzung zur Verwendung nach einem der Ansprüche 1 - 3, wobei in der Zusammensetzung zur einmaligen Verabreichung mindestens eine Menge von Blättern umfassend 0,460 mg 1,25-Dihydroxyvitamin-D3-Glycosid dem Tier als einmalige Dosis verabreicht wird.

5. Zusammensetzung zur Verwendung nach Anspruch 4, wobei die Menge an Blättern 0,550 mg 1,25-Dihydroxyvitamin-D3-Glycosid umfasst.

6. Zusammensetzung zur Verwendung nach einem der Ansprüche 1 bis 5, wobei ein Fettderivat ausgewählt wird und wobei das Fettderivat ein mindestens teilweise gehärtetes oder hydriertes Rizinusöl ist.

7. Zusammensetzung zur Verwendung nach einem der Ansprüche 1 - 6, wobei die Zusammensetzung zur Verabreichung mindestens 1 Tag vor dem Kalben, bevorzugt 1 oder 2 Tage vor dem Kalben bereitgestellt wird.

8. Zusammensetzung zur Verwendung nach einem der Ansprüche 1 - 7, wobei die Zusammensetzung des Weiteren einen oder mehrere Bestandteile umfasst ausgewählt aus der Gruppe bestehend aus Vitamin A, B12, C, E, K, Niacin, Thiamin, Cholin, Biotin, Folsäure, Riboflavin, Pantothensäure, einem oder mehreren Mineralien ausgewählt aus der Gruppe bestehend aus Kobalt, Kupfer, Mangan, Selen, Zink.

9. Zusammensetzung zur Verhinderung, durch Verabreichung als einmalige perorale Verabreichung, eines Symptoms ausgewählt unter mangelndem Appetit oder Anzeichen von Paralyse oder einer Kombination davon, das/die mit dem Jungen eines Tiers verbunden ist/sind, in einer Form, die für die perorale Verabreichung in Form einer Tablette oder eines Bolus geeignet ist, umfassend Blätter von Pflanzen mit, in diesen Blättern, mindestens 0,0460 mg 1,25-Dihydroxyvitamin-D3-Glycosid sowie eine Verbindung oder ein Material, die/das im Magen sich langsam abbaut oder körperlich erodiert wird, in welche Verbindung oder in welches Material die Blätter eingekapselt sind, wobei die Verbindung oder das Material aus der Gruppe ausgewählt werden bestehend aus einem Fett oder einem Derivat davon, einem Polysaccharid, einer Cellulose, einem Gummi, einem Glycol und Derivaten dieser Verbindungen, die für die verzögerte Freisetzung des 1,25-Dihydroxyvitamin-D3-Glycosids nützlich sind, wobei die Verbindungen, die die Freisetzung des 1,25-Dihydroxyvitamin-D3-Glycosids regulieren so ausgewählt werden, dass die Freisetzung während einer Zeitspanne von mindestens 3 Tagen fortdauert, und ein oder mehrere Metalle oder metallische Verbindungen, so dass die Zusammensetzung eine Masse/Volumen-Dichte erhält, die sie nach Verabreichung zum Absinken auf den Magenboden und dort Verbleiben bringt.

10. Zusammensetzung nach Anspruch 9, wobei die Blätter mindestens 0,550 mg 1,25-Dihydroxyvitamin-D3-Glycosid enthalten.

11. Zusammensetzung nach Anspruch 9 oder 10, wobei ein Fettderivat ausgewählt wird und wobei das Fettderivat ein mindestens teilweise gehärtetes oder hydriertes Rizinusöl ist.

12. Zusammensetzung nach einem der Ansprüche 9 - 11, wobei die Zusammensetzung in Form eines Bolus vorliegt und in einer Verpackung aufgenommen ist, die porös ist.

13. Zusammensetzung nach einem der Ansprüche 9 - 12, wobei die Zusammensetzung die Form eines Bolus aufweist.

14. Zusammensetzung nach einem der Ansprüche 9 - 13, wobei die Zusammensetzung des Weiteren mindestens ein Vitamin oder Mineral umfasst.

## Revendications

1. Composition contenant des feuilles provenant de plantes comprenant du glycoside de 1,25-dihydroxyvitamine D3, la composition étant pour une unique administration perorale sous la forme d'un comprimé ou d'un bolus, la composition comprenant :
- un composé ou une matière qui se décompose lentement ou s'érode physiquement dans l'estomac, dans lequel composé ou laquelle matière les feuilles sont encapsulées, en conséquence de quoi une libération retardée de glycoside de 1,25-dihydroxyvitamine D3 est effectuée, le composé ou la matière étant choisi dans un groupe consistant en une graisse ou un dérivé de celle-ci, un polysaccharide, une cellulose, une gomme, un glycol ou des dérivés de ces composés utiles pour la libération retardée du glycoside de 1,25-dihydroxyvitamine D3, les composés qui contrôlent la libération du glycoside de 1,25-dihydroxyvitamine D3 étant choisis pour que la libération continue pendant une période d'au moins 3 jours, et
- un ou plusieurs métaux ou composés métalliques afin que la composition obtienne un poids spécifique qui la fasse couler, après administration, au fond de l'estomac et y reste,
à utiliser, pour la prévention d'un symptôme choisi parmi moins d'appétit, une paralysie ou une combinaison des deux, chez un animal après une mise bas ou après un vélage, la composition devant être administrée peroralement comme une unique administration.

2. Composition à utiliser selon la revendication 1, dans laquelle les feuilles proviennent de Solanum glaucophyllum.

3. Composition à utiliser selon la revendication1 ou 2, la composition ayant une masse volumique d'au moins 1,5 kg/l.

4. Composition à utiliser selon l'une quelconque des revendications 1 - 3, la composition pour administration unique contenant au moins une quantité de feuille comprenant 0,460 mg de glycoside de 1,25 dihydroxyvitamine D3 qui est administrée à l'animal comme une dose unique.

5. Composition à utiliser selon la revendication 4, dans laquelle la quantité de feuilles comprend 0,550 mg de glycoside de 1,25 dihydroxyvitamine D3.

6. Composition à utiliser selon l'une quelconque des revendications 1 à 5, dans laquelle un dérivé de graisse est choisi et dans laquelle le dérivé de graisse est une huile de ricin au moins en partie durcie ou hydrogénée.

7. Composition à utiliser selon l'une quelconque des revendications 1 - 6, la composition étant prévue pour être administrée au moins 1 jour avant vélage, de préférence 1 ou 2 jours avant vélage.

8. Composition à utiliser selon l'une quelconque des revendications 1 - 7, la composition comprenant en outre un ou plusieurs composants choisis dans le groupe consistant en vitamine A, B12, C, E, K, niacine, thiamine, choline, biotine, acide folique, riboflavine, acide pantothénique, un ou plusieurs minéraux choisis dans le groupe consistant en cobalt, cuivre, manganèse, sélénium, zinc.

9. Composition pour prévenir par administration d'une unique administration perorale un symptôme sélectionné parmi moins d'appétit ou des signes de paralysie ou une combinaison de ceux-ci associé à la mise bas par un animal, sous une forme appropriée pour l'administration perorale sous la forme d'un comprimé ou d'un bolus, comprenant des feuilles de plantes avec, dans ces feuilles, au moins 0,460 mg de glycoside de 1,25-dihydroxyvitamine D3 ainsi qu'un composé ou une matière qui se décompose lentement ou s'érode physiquement dans l'estomac, dans lequel composé ou laquelle matière les feuilles sont encapsulées, le composé ou la matière étant choisi dans le groupe consistant en une graisse ou un dérivé de celle-ci, un polysaccharide, une cellulose, une gomme, un glycol ou des dérivés de ces composés utiles pour la libération retard du glycoside de 1,25-dihydroxyvitamine D3, les composés qui contrôlent la libération du glycoside de 1,25-dihydroxyvitamine D3 étant choisis pour que la libération continue pendant une période d'au moins 3 jours, et un ou plusieurs métaux ou composés métalliques afin que la composition obtienne un poids spécifique qui la fasse couler, après administration, au fond de l'estomac et y reste.

10. Composition selon la revendication 9 dans laquelle les feuilles contiennent au moins 0,550 mg de glycoside de 1,25 dihydroxyvitamine D3.

11. Composition selon la revendication 9 ou 10 dans laquelle un dérivé de graisse est choisi et dans laquelle le dérivé de graisse est une huile de ricin au moins en partie durcie ou hydrogénée.

12. Composition à utiliser selon l'une quelconque des revendications 9 - 11, la composition se présentant sous la forme d'un bolus et étant logée dans un emballage qui est poreux.

13. Composition à utiliser selon l'une quelconque des revendications 9 - 12, la composition se présentant sous la forme d'un bolus.

14. Composition à utiliser selon l'une quelconque des revendications 9 - 13, la composition comprenant en outre au moins une vitamine ou un minéral.
